# EUROPEAN PATENT APPLICATION

(11) **EP 0 566 783 A1**
(43) Date of publication of application: **27.10.1993**
(21) Application number: 92201107.7
(22) Date of filing: 21.04.1992
(51) Int. Cl.: C07C 209/02, C07C 211/56

(54) **Process for the production of nitrodiarylamines**

(71) Applicant: AKZO N.V., NL-6824 BM Arnhem (NL)
(72) Inventor: van Helden, Robert, NL-1422 JX Uithoorn (NL)
(74) Representative: Schalkwijk, Pieter Cornelis

(57) **Abstract**

A process for the production of nitrodiarylamines by the reaction of a nitroarene with an aromatic primary amine in a suitable solvent and in the presence of a base capable of abstracting a proton from said aromatic primary amine, is disclosed. The reaction is carried out at a reaction temperature of 20-70°C over a period of 6-24 hours and in a substantially oxygen-free atmosphere. The produced nitrodiaryl amines are useful as intermediates for the production of dyestuffs and rubber antidegradants.

## Description

The present invention relates to a method for the production of nitrodiarylamines which are useful as intermediates for the preparation of dyestuffs and rubber antidegradants. For example, 4-nitrodiphenylamine is used as an intermediate in preparing antiozonants useful in rubber compounds.

It is known to make nitrodiarylamines from nitrohaloarenes and N-acylaromatic amines or other activated forms of the amines in the presence of a so-called acid acceptor, for example, sodium carbonate. This process suffers from disadvantages since it requires large amounts of the acid acceptor and large quantities of by-products are formed.

An improved process is shown in U.S. patent 4,187,248, in which process a nitrohaloarene, such as p-nitrochlorobenzene is reacted with a sodium, potassium, rubidium or cesium salt of the formyl derivative of an aromatic primary amine, such as potassium formanilide. The process is a batch reaction, and is exothermic, and since all of the reactants are present in the reactor from the outset, temperature and the rate of the reaction are difficult to control.

European patent applications 0 147 379 and 0 148 145 relate to a process for producing nitrodiarylamines by combining the formyl derivative of an aromatic primary amine with a nitrohaloarene at the condensation temperature for forming nitrodiarylamines and adding an alkoxide or cycloalkoxide of sodium, potassium, rubidium or cesium. Also disclosed is that control of the rate of alkoxide addition serves to control the rate of the overall reaction, thus avoiding the possibility of excessive and dangerous temperature buildup in the reactor. However, this process suffers from the disadvantage that it requires the use of p-chloro-nitrobenzene as a starting material and the process for producing p-chloro-nitrobenzene also produces a significant amount of the by-product o-chloro-nitrobenzene. Further, the aminoarene must be protected by reaction with a formyl group in order to carry out this reaction.

The present inventors have surprisingly found that nitroarylamines can be obtained directly via a condensation of nitroarene and an aromatic primary amine to produce a high selectivity for the nitroarylamine and a low quantity of undesirable by-products.

Accordingly, the present invention provides a process for the production of nitrodiarylamines by reacting, in a polar aprotic solvent and in the absence of oxygen, a nitroarene with an aromatic primary amine in the presence of a base capable of abstracting a proton from the aromatic primary amine. The reaction is carried out at a condensation temperature suitable for forming nitrodiarylamine. In this manner, nitrodiarylamines can be obtained in high yield with a high selectivity.

The process of the present invention is a general process for the condensation of nitroarenes with aromatic primary amines. As examples of typical aromatic primary amines which may be used in the process are included aniline, benzidines, paraphenylene diamine, aromatic polyamines, amino pyridines, aniline derivatives which may be substituted with one or more alkyl , alkoxy, nitro, fluoro or chloro substituents and other amino heterocyclic compounds such as thiophenes and pyrroles. For example, o-methylaniline, o-methoxyaniline, o-ethylaniline, o-isopropylaniline, o-butylaniline, m-chloroaniline, m-fluoroaniline, α-naphthyl amine, β-naphthyl amine and o-nitroaniline may be employed as the aromatic primary amines.

A variety of nitroarenes are suitable for use in the process of the present invention. Illustrative of such nitroarenes are nitrobenzene, o-nitromethylbenzene, o-nitroethylbenzene, 3,5-dimethylnitrobenzene, 3,5-diethylnitrobenzene, m-nitrotoluene, o-nitrotoluene, dinitrotoluene, dinitrobenzene and m-dinitrotoluene, among others.

Generally, it is preferred to use a mole ratio of 1 to 3 moles of amine moieties of aromatic primary amine to each mole of nitroarene, and more preferably a ratio of 1 to 1.5 moles of amine moieties of aromatic primary amine to each mole of nitroarene. By amine moieties is meant that, in the case of using a polyamine, each amino group in that polyamine counts as a separate amine moiety. Thus, for example, 1 mole of amine moieties per mole of nitroarene, in the case of a diamine, refers to 0.5 moles of diamine per mole of nitroarene.

The process of the present invention is carried out in the presence of a base capable of abstracting a proton from the aromatic primary amine. The abstraction of the proton from the aromatic primary amine is believed to be the rate-determining step in the reaction. Each base will reach an equilibrium with the aromatic primary amine and, due to the consumption of amine which has had its proton extracted, by reaction with the nitroarene, additional proton extraction will occur in order to maintain the equilibrium. Accordingly, it is possible to control the rate of the reaction by selection of an appropriate base on the basis of its proton-abstraction equilibrium constant with the particular aromatic primary amine to be reacted.

The base may be selected from compounds such as alkali metal and alkali earth metal hydroxides, alkoxides, amides and hydrides, as well as salts of alkali metals and alkali earth metals and appropriate polar aprotic solvents. Examples of suitable bases include potassium 2-butoxide, potassium hydroxide, potassium 2-methyl-2-butoxide, sodium 2-methyl-2-butoxide, potassium isopropoxide, potassium 2-octyloxide, potassium t-butoxide, potassium n-butoxide, sodium methoxide, potassium methoxide, potassium 2-pentanoxide, potassium 3-methyl-1-butoxide, potassium 1-pentanoxide and solutions of sodium or potassium in dimethyl sulfoxide. The preferred bases are potassium hydroxide, potassium t-butoxide and a sodium salt of dimethyl sulfoxide.

The amount of base employed should be from 0.1 to 3.0 moles of base per mole of amine moieties of aromatic primary amine and more preferably from 0.7 to 1.0 moles of base per mole of amine moiety of aromatic primary amine.

The base may be added neat to the reaction, but solid bases such as potassium hydroxide and potassium t-butoxide, are more easily and conveniently added in the form of a solution in an appropriate polar aprotic solvent. Any polar aprotic solvent in which the bases are soluble may be used, but it is preferred to use sulfoxides, sulfolanes and alkoxy-containing solvents. More particularly, useful solvents include dimethoxy sulfoxide, sulfolane, dimethoxy ethane, dimethyl formamide, dimethyl acetamide, N-methyl pyrrolidone and tetrahydrofuran, among others. Dimethyl sulfoxide is the most preferred solvent. Sufficient solvent must be employed to solubilize the entire reaction mixture including the base, the aromatic primary amine and the nitroarene.

In some circumstances it may be desirable to add a second solvent, which need not be a polar aprotic solvent, to the reaction mixture in order to enhance the dissolution of the aromatic primary amine in the reaction mixture. Suitable solvents include those which dissolve aromatic primary amines. Most preferred are alcohols such as t-butanol and methanol.

In the process, the reactants may be added in any order. The reaction must be carried out at a temperature which is suitable as a condensation temperature for forming nitrodiarylamines. More particularly, preferred reaction temperatures will range from 20 to 70°C and more preferably will be between 20 and 50°C. The preferred process of the invention is performed under agitation to increase contact among the reactants. The reaction time will vary from 6-24 hours.

The reaction must be carried out in an oxygen-free atmosphere in order to prevent undesirable side reactions caused by oxidation. Accordingly, the reaction may be carried out in a nitrogen or inert gas atmosphere.

The reaction may be carried out in steel, stainless steel, glass or glass-lined reaction vessels. After the reaction is complete, the impurities may be removed by distillation in, for example, a film evaporator and the products may be purified by refluxing with n-hexane, or more preferably, by crystallization from carbon tetrachloride and filtering.

It is important to note that before employing purification processes involving temperatures in excess of 70°C, the remaining unreacted base should be neutralized to prevent base-catalyzed side reactions during the purification that can lead to undesirable by-products. Weak acids such as acetic acid are preferred neutralizing agents since their use prevents the creation of a strongly acidic environment. Stronger acids may also be used in smaller quantities, if desirable.

Optionally, a small quantity of a phase transfer catalyst may be added to the reaction mixture in order to help solubilize the base and thereby create a more basic reaction system. An example of such a phase transfer catalyst is tetrabutylammonium hydrosulfate. This catalyst is added in a conventional, known quantity which is used for phase transfer catalysts.

Also, under some conditions, the reaction of the base with the aromatic primary amine will produce water or another diluent. This tends to reduce the overall basicity of the reaction mixture and thereby slow the reaction. If such conditions exist, it may be useful to add a water-absorbing agent, such as sodium sulfate, to the reaction mixture in order to maintain the strong basicity of the solution despite the formation of water during the reaction.

The following examples are presented to further illustrate the present invention.

### Example 1

Reaction of nitrobenzene, aniline and potassium t-butoxide in a dimethyl sulfoxide (DMSO)/t-butanol solvent.

To a well-stirred, cooled solution of 17.0 g. (0.182 mol) aniline in 80 ml DMSO is added 13.5 g. potassium t-butoxide (0.12 mol) and 12.5 ml t-butanol while nitrogen is passed through at temperatures less than 20°C. Then, 22.8 g. nitro-benzene is added (0.185 mol) followed by 10 ml DMSO. The temperature is maintained at 20-25°C. The mixture is stirred for 6.75 hours at 20°C and the reaction stopped by adding 8 ml acetic acid and 8 ml water. The product is distilled in a film-evaporator at 20 mm Hg/100°C. The distillate consists of DMSO, t-butanol, water and the majority of the remaining unconverted nitrobenzene and aniline as shown by NMR analysis.

The residue (59.7 g.) is diluted with water (to dissolve K-acetate and small amounts of DMSO which may remain) and n-hexane while stirring. The crystalline dark-yellow product obtained is collected by filtration, washed with water and hexane, and dried. This gives 19.1 g. of crude p-nitrodiphenyl amine.

The crude p-nitrodiphenyl amine is refluxed with hexane, cooled to room temperature and filtered, yielding 18.1 g. of para-nitrodiphenyl amine, containing about 10% phenazine and phenazine precursor, as confirmed by NMR analysis. From the combined hexane filtrates, 3.9 g. of a mixture of nitrobenzene, aniline and phenazine is obtained. In total, 0.076 mol p-nitrodiphenyl amine (selectivity 82%) and 0.017 mol phenazine (selectivity 18%) are obtained.

The crude product can be purified by crystallization from carbon tetrachloride and filtering. From the filtrate, phenazine is obtained. The crude product can also be purified by extraction of a methylene chloride solution with 10% hydrochloric acid. Upon neutralization of the hydrochloric acid extract with sodium hydroxide, phenazine and phenazine precursor can be collected by filtration. Reduction of this mixture with Zn/hydrochloric acid in methanol yields phenazine.

### Examples 2-3

Example 1 was repeated using molar ratios of nitrobenzene/aniline/potassium t-butoxide of 2/1/0.7 and 1/2/0.7 and about the same results as in example 1 were obtained.

### Example 4

Example 1 was repeated except that the reaction was carried out over a 24 hour period. A 93% yield on the basis of potassium t-butoxide was obtained with a selectivity of 74% for p-nitrodiphenyl amine.

### Example 5

Example 1 was repeated except that the reaction was carried out at 50°C over a period of 6 hours. A 90% yield on the basis of potassium t-butoxide was obtained with a selectivity of 79% for p-nitrodiphenyl amine.

### Example 6

Example 1 was repeated except that the neutralization with acetic acid was omitted from the procedure. In this instance the results were substantially the same except that, in addition, azobenzene was obtained as a by-product.

### Example 7

Example 1 was repeated except that the solvent was DMSO alone. Approximately the same results were obtained as when the solvent comprised DMSO and t-butanol.

### Example 8

Example 1 was repeated using a solvent comprising sulfolane and t-butanol in a volume ratio of 7/2. Products were obtained in a 50% yield on the basis of potassium t-butoxide after a reaction period of 24 hours at 23°C. The products exhibited a selectivity of 75% for p-nitrodiphenyl amine.

### Example 9

Example 1 was repeated using o-toluidine in place of aniline and employing a molecular ratio of nitrobenzene/o-toluidine/potassium t-butoxide of 1/1/0.6, 4-nitro-2'-methyl-diphenyl amine was obtained with a selectivity of 80%.

### Example 10

To a stirred solution of 17.0 g. aniline, 22.8 g. nitrobenzene in 150 ml DMSO and 21 ml t-butanol, were added 7.0 g. of freshly powdered anhydrous potassium hydroxide and 0.25 g. NBu₄HSO₄ was added at 22°C while nitrogen was passed through the reaction zone. The mixture was reacted for 22.5 hours at 20-25°C and then neutralized with 8 ml acetic acid and 8 ml water. The same purification procedures as used in example 1 were then employed and a yield of 29% on potassium hydroxide was obtained with a selectivity of greater than 95% for p-nitrodiphenyl amine.

### Example 11

Example 10 was repeated using only 60% of the amount of solvent. A yield of 24% on the basis of potassium hydroxide was obtained with a selectivity of greater than 95%. When the reaction product was kept for several days at room temperature before neutralization, a 41% yield resulted.

### Example 12

Example 10 was repeated omitting the t-butanol. Approximately the same results were obtained except with a slightly lower yield.

### Example 13

The reaction was carried out using 10 g. of aniline, 16.8 g. of nitrobenzene, 5.4 g. of powdered potassium hydroxide and 10 g. of sodium sulfate in 50 ml of dimethyl sulfoxide solvent at 50-55°C over a period of 24 hours while passing nitrogen through the reaction zone. The crude product (9.0 g.) was crystallized from carbon tetrachloride yielding 8.0 g. of pure p-nitro diphenylamine (dark yellow needles), a 37% molar yield based on potassium hydroxide.

### Example 14

Example 13 was repeated with the addition of 0.3 g. of tetrabutyl ammonium hydrosulfate phase transfer catalyst. Virtually the same results were obtained except the reaction was somewhat faster.

### Example 15

Example 13 was repeated omitting sodium sulfate. This gave, after crystallization from carbon tetrachloride, 6.0 g. of p-nitro diphenylamine, for a 30% molar yield based on potassium hydroxide.

### Example 16

To a stirred solution of 10 g. of aniline in 50 ml of dimethyl sulfoxide, 2.3 g. of sodium wire (washed first with petroleum ether) was added while nitrogen was passed through the reaction zone. The mixture was heated to 50-60°C for 2 hours. Hydrogen gas was evolved and the sodium metal slowly dissolved in the solvent. Then, the mixture was heated to 115°C for 15 minutes to complete the sodium dissolution. After cooling to 32°C, 16.8 g. of nitrobenzene is added and the mixture reacted for 19½ hours at 50-55°C. Neutralization with acetic acid and crystallization from carbon tetrachloride yielded 7.2 g. of crude p-nitro diphenylamine.

### Example 17

To a stirred solution of 5.2 g. of p-phenylene diamine in 75 ml. of dimethyl sulfoxide were added 11.0 g. of potassium t-butylate while passing nitrogen through the reaction zone. Then, 18.0 g. of nitrobenzene is added and the mixture reacted for 5½ hours at 23-28°C. Neutralization with acetic acid followed by crystallization from carbon tetrachloride yielded 11.0 g. of crude p-nitro, p-amino-diphenyl amine.

The foregoing examples were presented for the purpose of illustration and description only and are not to be construed as limiting the scope of the invention in any way. The scope of the invention is to be determined by the claims appended hereto.

## Claims

1. A process for the production of nitrodiarylamines which comprises reacting, in a polar aprotic solvent, a nitroarene with an aromatic primary amine in the presence of a base capable of abstracting a proton from said aromatic primary amine, at a condensation temperature suitable for forming nitrodiarylamines and in a substantially oxygen-free atmosphere to obtain at least one crude nitrodiarylamine.

2. A process in accordance with claim 1 wherein one to three moles of amine moieties of said aromatic primary amine are employed for each mole of nitroarene.

3. A process in accordance with any one of claims 1-2 wherein said base is employed in an amount of from 0.1 to 3.0 moles per mole of amine moieties of said aromatic primary amine.

4. A process in accordance with any one of claims 1-3 wherein said condensation temperature is from 20 to 70°C and said reaction is carried out over a period of 6-24 hours.

5. A process in accordance with any one of claims 1-4 wherein said base comprises at least one alkali metal or alkali earth metal salt of hydroxides, alkoxides, amides and hydrides, or a salt of at least one alkali or alkali earth metal and at least one polar aprotic solvent.

6. A process in accordance with any one of claims 1-5 wherein said solvent is selected from sulfoxides, sulfolanes, alkoxy-containing solvents, tetrahydrofuran, dimethyl formamide, dimethyl acetamide and n-methyl pyrrolidone.

7. A process in accordance with any one of claims 1-6 wherein said nitroarene is nitrobenzene and said aromatic primary amine is aniline.

8. A process in accordance with any one of claims 1-7 further comprising the step of purifying said at least one crude nitrodiarylamine by distillating said crude nitrodiarylamine, separating the distillate and recrystallizing the distillation residue from carbon tetrachloride.

9. A process in accordance with claim 8 further comprising the step of neutralizing the remaining base present in said crude nitrodiarylamine with an acid, prior to said distillation step.

10. A process in accordance with any one of claims 1-9 wherein said reaction is carried out in the presence of an effective amount of a phase transfer catalyst.

11. A process in accordance with any one of claims 1-10 wherein said reaction is carried out in the presence of an effective amount of a water-absorbing agent.
